# EUROPEAN PATENT SPECIFICATION

(11) **EP 2 853 291 B1**
(45) Date of publication and mention of the grant of the patent: **10.08.2016**
(21) Application number: 13186294.8
(22) Date of filing: 27.09.2013
(51) Int. Cl.: A61N 5/06

(54) **Irradiating device having high evenness of irraditation and even irradiation method**
Bestrahlungsvorrichtung mit hoher Gleichmäßigkeit von Bestrahlung und gleichmäßiges Bestrahlungsverfahren
Dispositif d'irradiation ayant une uniformité élevée d'irradiation et procédé d'irradiation uniforme

(43) Date of publication of application: 01.04.2015
(73) Proprietor: JK-Holding GmbH, 53578 Windhagen (DE)
(72) Inventor: Gerstenmeier, Jürgen, 53578 Windhagen (DE)
(74) Representative: Schwabe - Sandmair - Marx

(56) References cited:
- DE-A1- 4 026 327
- DE-A1- 10 233 984
- DE-U1- 9 419 787
- FR-A1- 2 598 921
- US-A1- 2012 150 265

## Description

The present invention relates to a device allowing an even application of actinic radiation to a subject, and relates to a non-therapeutical method of allowing an even application of actinic radiation to a subject. In particular, the present invention relates to a device and non-therapeutical method allowing an even application of tanning or activating or curing actinic radiation to a subject as, for example, to a human being.

Devices and methods of applying actinic radiation to a subject, for example to a human being, are widely known since a long time and are practically used for a number of purposes.

Tanning of the human body, i. e. the activation of melanin generation and melanin conversion into the dark (brown) form of the pigment naturally occurring in the human skin, is effected by the application, to the skin of a subject, e. g. to the skin of a human being, of actinic radiation having a wavelength λ in the range of from 280 to 315 nm (UV-B radiation) and/or of actinic radiation having a wavelength λ in the range of from 315 to 400 nm (UV-A radiation). Tanning may serve purposes in the cosmetic and/or medical field(s), and the number of applications in either of these fields is steadily increasing; see document EP-A 1 738 797 filed in the name of the present applicant, and the document DE-A 10 2005 063 156.

Moreover, short wavelength UV-B radiation in the wavelength range of λ = 297 nm ± 10 nm is well known to promote Vitamin D biosynthesis from Vitamin D precursors in the human skin; see the document WO-A 2005/000 389.

It was found recently that actinic radiation having wavelengths λ in the visible and near IR (infrared) range (400 or, even better, > 550 nm to 850 nm), when applied to the human skin, is capable of activating the biosynthesis of beneficial compounds for the skin's nourishing and rejuvenation, as for example elastin, keratin and hyaluronic acid; see document EP-A 2 500 060 filed in the name of the present applicant; and the document WO-A 2010/070,277.

The document US 2012/150,265 A1 relates to a red and near infra-red light phototherapy apparatus designed to accommodate an adult user. A user support of the device may have a hingably connected canopy that contains the red, and infra-red, light source. When the canopy is open, a user may lie on the user support. When adjusted to an operational position, the upper light source may illuminate the entire user support. The apparatus may include an acrylic support with a lower red, and infra-red, light source attached beneath. The acrylic support may be transparent to the red, and infrared, light. The lower light source may illuminate the entire acrylic support from below.

The document DE102 33 984 A1 relates to a solarium which comprises two tunnel-shaped units fitted with UV-lamps and optionally HPA-lamps for tanning the face. It can be used in the vertical or horizontal position.

The document DE 40 26 327 A1 discloses a treatment apparatus which comprises a couch surface and an upper radiation element. At least one selectively switchable UB radiation source is assigned to at least the upper radiation element. At least one colour light source is provided in the latter for irradiating the user lying on the couch section. A lower radiation element can be provided underneath the couch area. At least one colour light source is also provided, which colour light source can be in the form of a fluorescent tube to extend over the whole length of the radiation elements or only part of it.

The document FR 2 598 921 A1 relates to a phototherapy device and method intended for the emission of light energy in the direction of the skin of a patient, with a view to phototherapeutic or photochemotherapeutic treatment of dermatoses. The device comprises several tubes or groups of tubes for emitting radiation of different types (UVA, UVB, actinic rays, etc.), and it is fitted with means for programming the start time and duration of emission of each tube or each group of tubes during the same phototherapy session. It is thus possible to modulate the energy emitted, and its emission time, during a session, as a function of the total time programmed for that session.

The document DE 94 19 787 U1 relates to an irradiation couch having a canopy positioned above a longitudinal rest area and containing longitudinal irradiation light sources which extend at least across a part of the rest area transvers to the longitudinal rest area.

While proposals were made recently that the usual actinic radiation application devices allowing the application of actinic radiation to the user or subject, particularly to a human being, in his/her recumbent position, i. e. lying more or less horizontally on a so-called "tanning bed", be replaced or supplemented by devices allowing the application of actinic radiation to the user or subject in a standing or vertical position, the latter proposal could not result into a change of the user's habits to receive the actinic radiation in the recumbent position due to the fact that this is considered to be more convenient and relaxing. Hence, the bed-like devices attracted more attention of the users and, hence, were further improved in view of their safety and comfort features as well as in view of an even better result of the respective actinic radiation applied to the user for the above (and other) purposes.

One of the disadvantages observed by many users was the fact that not all areas of the body of the user could receive the actinic radiation to which the user/subject was exposed in an even manner. This was considered to be the result of the fact that the user's body, when exposed to actinic radiation on tanning beds (or bed-like devices) in a recumbent position, had to lie in a so-called "exposition tunnel". Such an exposition tunnel is formed by a flat lower surface made, for example, from an acrylic polymer and substantially permeable for the desired actinic radiation emitted by actinic light radiation-emitting sources mounted below said surface, and a semi-cylinder barrel-shape upper surface usually made of the same material as the lower surface and, hence, also substantially permeable for the desired actinic radiation emitted by actinic light radiation-emitting sources mounted above said barrel-shape surface. Since not all points of the user's body surface are in one plane, and since not all actinic radiation emitting light sources are arranged in one plane, the distance of the radiation-emitting light sources to the user's body surface which has to receive the actinic radiation is different for almost all points on the user's body surface.

Since the radiant flux (or radiant power) φ of all actinic radiation-emitting light sources emitting the same type of radiation was the same in the prior art, the irradiance E (i. e. the amount of actinic radiation received by the user's body) was different at each point of the user body's surface, due to the different distances the radiation had to "travel" from the light source(s) to the user's body. Hence, the effect of the respective actinic radiation (for example and without restriction: the tanning result in cases of UV-A and UV-B radiation, or the vitamin B-production in cases of UV-B radiation in the range needed, or the keratinogenetic effect of visible red and/or near infrared (IR) radiation) was different and, in view of the user's desire, was sometimes insufficient.

Moreover, the above consideration is based on the assumption that each actinic radiation-emitting light source is a point-shaped light source emitting the actinic radiation from one "geometric" spot. This is, however, a theoretical assumption. Practically, the actinic radiation-emitting light sources have a cylinder shape having a section similar to a sphere and are provided with a reflector on the inner concave cylinder surface or outside of the cylinder for shaping and/or concentrating the radiation emitted. In addition, the radiation emitted by the light sources has to pass a filter, in some cases, thus making the radiation emitted even more non-point or even diffuse.

The above disadvantage should be overcome. Hence, it was an object of the present invention that the radiant intensity of all actinic radiation-emitting light sources be controlled, and varied in such a manner that an even irradiance be achieved over the whole body surface of the user. Thereby, the efficiency of irradiation devices and of the irradiation method should be enhanced, and over-exposures should be prevented.

Surprisingly, the above disadvantage could be overcome, and an even irradiance could be achieved over the user's whole body surface, by an adjustment of the radiant flux (or radiant power) φ of the actinic light-emitting light sources to the geometric parameters of the irradiation device.

Hence, the invention relates to a device for an application of directed actinic radiation to a subject, said device comprising:
- an exposition tunnel being capable of surrounding said subject to be exposed to said actinic radiation, said exposition tunnel being formed of one semi-cylinder barrel-shape first surface made of a material substantially permeable to said actinic radiation to be applied to the subject, and of at least one second surface made of said material substantially permeable to said actinic radiation to be applied to the subject, said at least one second surface being capable of complementing the first surface in forming said exposition tunnel;
- the surfaces of said exposition tunnel separating an inner space where a subject is exposed to said actinic radiation, from outer spaces where light sources capable of emitting actinic radiation through said surfaces are mounted;
- said outer spaces being housed in at least first and second parts of a chassis of the device and comprise means for fixedly holding, and electrically and electronically operating, said light sources; means providing said at least first and second parts of the chassis with electric power; means capable of heating and/or cooling the device; and means for the operation of the device, including a processing unit;
said device further comprising
at least one means capable of controlling a radiant flux φ of at least one actinic radiation-emitting light source by changing the at least one light source's electronic parameters in accordance with the geometric position of said at least one actinic radiation-emitting light source, relative to its distance from the inner space, thereby achieving an even radiance E at the subject exposed to said actinic radiation, wherein said means capable of controlling, and optionally varying, a radiant flux φ of at least one actinic radiation-emitting light source (170) comprise at least one or more than one electronic control member (171) changing the light sources' electronic parameters in accordance with the geometric position of said at least one actinic radiation-emitting light source (170), relative to its distance from the inner space (130).

Preferred embodiments of the device of the invention are claimed in dependent claims 2 to 11.

The invention further relates to a non-therapeutical method for operating a device applying a directed actinic radiation to a subject, said process comprising the steps of: providing a device as described in further detail in the subsequent specification; and operating at least one of the device's actinic light radiation-emitting light sources by controlling at least a radiant flux φ of at least one of said actinic radiation-emitting light sources by changing at least one of its electronic parameters in accordance with the geometric position of said at least one actinic radiation-emitting light source, relative to its distance from the inner space of the device, thereby achieving an even radiance E at exposure to said actinic radiation emitted by said light source.

Preferred embodiments of the method are claimed in the dependent claims 13 to 15.

The present invention is now further described in detail by referring to Figures, wherein
Figure 1 shows an embodiment of the device of the invention for an application of directed actinic radiation to a subject in the form allowing a recumbent position of the subject when exposed to said radiation;
Figure 2 shows another embodiment of the device of the invention for an application of directed actinic radiation to a subject in the form allowing an erected position of the subject when exposed to said radiation;
Figure 3 shows two embodiments of actinic radiation-emitting light sources being a component of the device of the invention for an application of directed actinic radiation to a subject; and
Figure 4 shows the geometric relations in the device for an application of directed actinic radiation as shown in Figure 1.

Reference is now made, for a detailed description of the invention, as broadly defined, and of its particular, in part preferred, embodiments, to the above Figures. In the following description, a reference to preferred embodiments, either in the description or in the Figures, should not be construed as a restriction of the invention to the preferred embodiments. Preferred embodiments, even if described in the description as such or shown in one or more than one of the Figures, serve only the purpose of exemplarily demonstrate the invention for a better understanding thereof. The scope of the invention can be derived by a skilled person from the claims.

The terms "comprise", "comprises" or "comprising" as used in the present specification and claims, for example in claim 1 or in claim 12, has/have the meaning that the device of the invention may comprise (i) one means or may comprise (ii) two or more means as mentioned in, for example, claim 1, or that (iii) further components, means etc. (more specifically defined below) may also be comprised by the device. In a similar manner, the terms "comprise", "comprises" or "comprising" has/have the meaning that the non-therapeutical method of the invention may comprise (i) one step or may comprise (ii) two or more steps as mentioned in, for example, claim 12, or that (iii) further steps etc. (more specifically defined below) may also be comprised by the method.

The terms "comprise" , "comprises" or "comprising" as used in the present specification and claims may, however, also include cases where the device/non-therapeutical method of the invention mainly consists of (i) at least one means/step or mainly consists of (ii) two or more means (or steps) mentioned, for example, in claim 1 / claim 12, optionally together with any necessary component or means or step a skilled person may include into such a device / non-therapeutical method in order to achieve the object of the invention, or may even include cases where the device / non-therapeutical method of the invention exclusively consists of (i) at least means or step or exclusively consists of (ii) two or more means or steps, optionally, but not necessarily, together with any necessary component, means, step etc. a skilled person may include into such a device / non-therapeutical method in order to achieve the object of the invention. Particularly in the latter case where the terms "comprise" , "comprises" or "comprising" as used in the present specification and claims may have the meaning of an "exclusively consisting of", subclaims of the present application may claim, and corresponding parts of the specification may describe, further preferred embodiments, which are characterized by additional specified features which, in combination with the features of the independent claim and corresponding parts of the description, are summarized to belong to the invention as described in its broadest scope claimed.

In other words: The terms "comprise" or "comprises" or "comprising" may have, in the present specification and claims, the meaning of describing a non-exhaustive enumeration of elements / steps or, alternatively, may have, in the present specification and claims, the meaning of describing an exhaustive enumeration of elements / steps, in the latter case without excluding further preferred embodiments being characterized by additional features.

Hence, by the terms "comprises", "comprise" or "comprising", a non-exhaustive enumeration and an exhaustive enumeration of elements or features or method steps is disclosed in the present application and claimed in the claims 1 to 15.

The features according to the present specification described in connection to the preferred embodiments may each be realized as single features or may be realized in combination with one further feature or together with few other features or with several other features or with all features described in the present specification and claimed in the claims. All these combinations of two or more features are coved by the present invention as claimed.

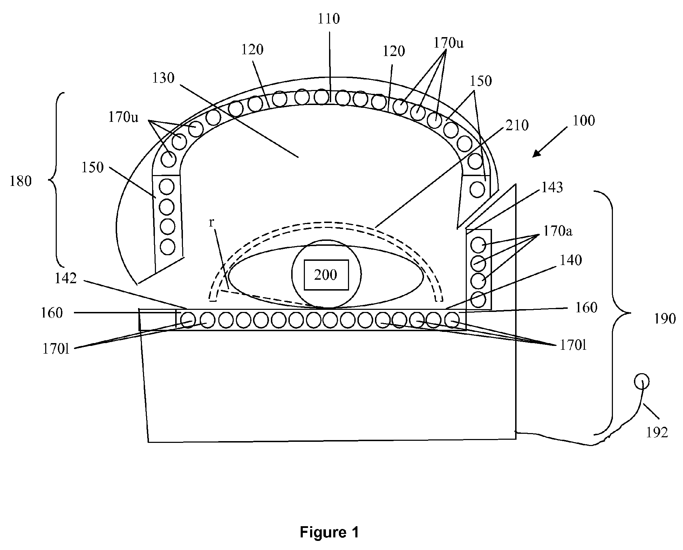

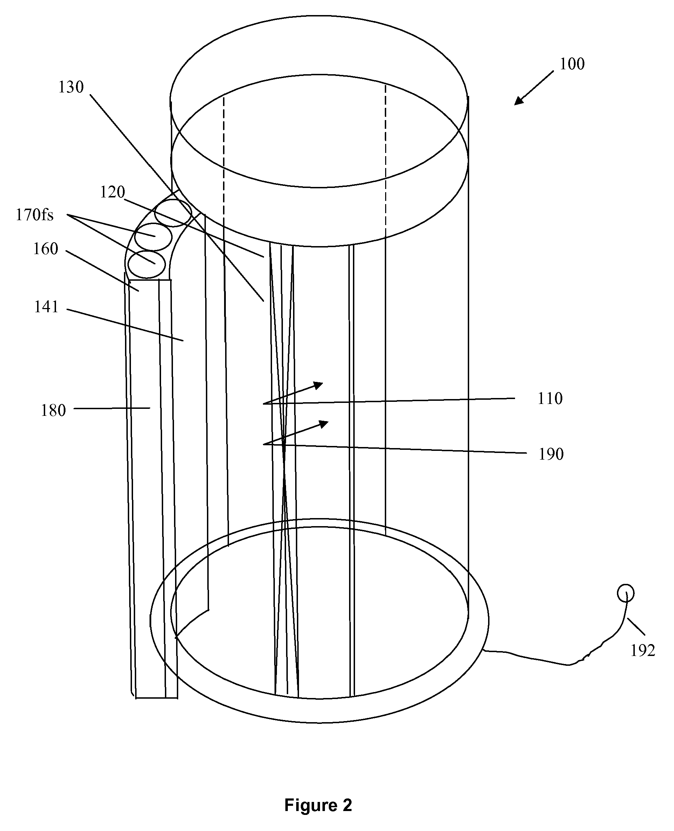

Reference is now made to Figures 1 and 2 showing two preferred embodiments of a device 100 of the present invention.

Such a device 100 as shown in Figures 1 or 2 serves an application, to a subject 200, of directed actinic radiation.

The term "actinic radiation", as used in the present specification and claims, is understood to mean light or (broader) radiation of the whole electromagnetic spectrum (see the definition given in "Römpp Chemie-Lexikon", Thieme Publishers, Stuttgart, Germany), which has a photochemical (including a photobiochemical) effect and includes, as the case may be, light/radiation of natural or artificial origin. In the claims and specification, "actinic light" or "actinic radiation" is mainly, but not restrictively, used for light or radiation having an artificial origin, e. g. light/radiation emitted by light sources in a device of the present invention.

In one preferred embodiment of the device 100 of the invention, which may be realized separately or together with one or two or several or all other features of the invention, said actinic radiation may be broad wavelength range actinic radiation. Alternatively, albeit also preferred, said actinic radiation may be narrow wavelength range actinic radiation or even actinic radiation of a very specific wavelength or several specific wavelengths. This is known to a skilled person, who may select the wavelength or wavelengths or wavelength bands to be applied in accordance with the requirements of a specific case. Particular examples of actinic radiation having useable wavelengths are: actinic radiation having a wavelength λ in the range of from 280 to 315 nm (UV-B radiation) and/or of actinic radiation having a wavelength λ in the range of from 315 to 400 nm (UV-A radiation) for tanning the human body for the activation of melanin generation and melanin conversion into the dark (brown) form of the pigment naturally occurring in the human skin, which tanning may serve purposes in the cosmetic and/or medical field(s); actinic radiation in the form of short wavelength UV-B radiation in the wavelength range of λ = 297 nm ± 10 nm for promoting Vitamin D biosynthesis from Vitamin D precursors in the human skin; or actinic radiation having wavelengths λ in the visible and near IR (infrared) range (400 or, even better > 550 nm, to 850 nm), which actinic radiation, when applied to the human skin, is capable of activating the biosynthesis of beneficial compounds for the skin's nourishing and rejuvenation, as for example elastin, keratin and hyaluronic acid. These examples, however, should not be construed to restrict the invention.

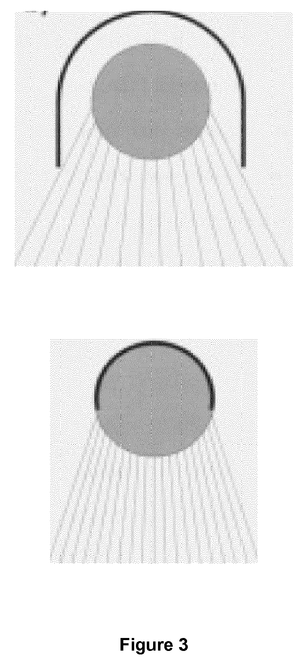

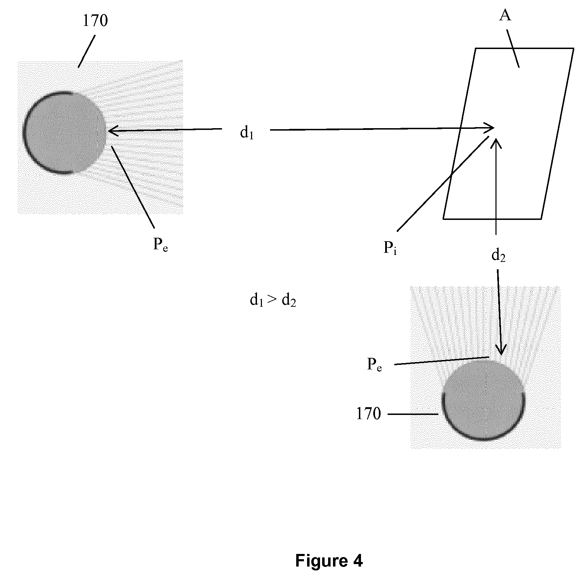

The term "directed" in combination with "actinic radiation", as used in the present specification and claims, is understood to mean actinic radiation which is emitted by an actinic radiation-emitting light source and which has a direction originating from a light source and arriving at, or impinging on, a certain target, e. g. a (general) surface A or a (specific) body surface of a subject/user 200. This is shown in Figures 3 and 4. As an example, Figure 3 shows a light source with a reflector and emitting actinic radiation. In the frame of the present invention, the target preferably is a subject, particularly a human being. Directed radiation may be emitted from light sources provided with shutters, reflectors, filters, other directing means or combinations thereof.

The term "actinic light-emitting light source" or, simply "light source", as used in the present specification and claims, is understood to mean means emitting light/radiation of a certain wavelength or of a more or less broad wavelength band. In accordance with the invention, any such light-emitting means may be designated to be an actinic light-emitting light source. A skilled person knows a great number of different light sources in the frame of the above definition. Exemplary embodiments of such actinic light-emitting light sources are low pressure light emission lamps (or tubes) or high-pressure light emission lamps (or radiators), which usually emit light of relatively broad wavelength bands, and of which various types are well-known to a skilled person and need no more detailed description here. The term "actinic light-emitting light source" also includes LED's which usually emit light of very specific (and usually narrow) wavelength bands. All such actinic radiation-emitting light sources may be used in the present invention, as a skilled person will easily learn from the subsequent specification and from the claims.

The term "subject", as used in the present specification and claims, is understood to mean any subject to which actinic radiation can be applied in a directed manner. A skilled person knows a number of subjects (including apparatus) to which actinic radiation may be applied. In preferred embodiments of the invention, the subject is a human being, and the terms "human", "human being" and "subject" are used synonymously in the present application.

The terms "application" and "apply" and "applying", as used in the present specification and claims, are understood to mean, when combined with "actinic radiation" or (more generally) "radiation", the step of irradiating actinic radiation from an actinic radiation-emitting light source to a subject with the aim of exerting a certain photochemical or photobiochemical effect.

The term "even" or "evenness", as used in the present specification and claims, is understood to mean that actinic radiation emitted by controlled radiant flux light sources in accordance with the invention is capable of resulting, ideally at each point of the subject to be exposed to said actinic radiation in a directed manner, into the same "amount" of a photochemical or photobiochemical effect due to the same radiance E at said subject, independent upon the geometrical conditions, e. g. upon the distance the actinic radiation has to "travel" from the emission point by the light source to the target point at the "surface" of the subject and upon the shape of the light source (which usually cannot have the ideal point shape) and upon the shape of the subject to be exposed to said radiation. An even radiance E at the subject to be exposed to directed actinic radiation was the object to be achieved by the present invention. A similar depth of tanning, or a similar amount of photosynthesized Vitamin D or a similar amount of photobiochemically generated keratin in a standard area of the users skin can be considered as (non-restricting) examples of the term "same amounts of a photochemical or photobiochemical effect".

In accordance with the present invention, the device 100 for an application of directed actinic radiation to a subject 200 comprises several elements which are explained in detail below.

Such a device 100 may be a well-known device with respect to the majority of its elements and is described in detail generally and in its preferred embodiments, by referring to Figures 1 and 2, and in particular to Figure 1, as follows:
The device 100 for an application of directed actinic radiation to a subject 200 comprises, as its central element, a so-called "exposition tunnel" 110. The term "exposition tunnel", as used in the present specification and claims, is understood to mean a tunnel-shaped space 110 wherein a subject 200 can be received, i. e. which is capable of surrounding said subject 200, and wherein said subject, when received, may be exposed to directed actinic radiation. The size and shape of such an exposition tunnel 110 is not restricted and may be selected by a skilled person in accordance with the requirements of the specific case. A skilled person knows from the general art published in this technical field the size and shape of an exposition tunnel 110 capable of surrounding a subject for the application of actinic radiation. In preferred embodiments of the invention (as in the prior art), the exposition tunnel may have a size suitable to house the subject in a convenient (lying, i. e. recumbent, or standing, i. e. erect) position. Hence, the size may be from 200 to 220 cm in length, 90 to 120 cm in width around said subject, without restricting the invention to such a size. In further preferred embodiments of the invention (not restricting the invention), the shape of an exposition tunnel 110 is an (at least approximate) cylinder shape.

In one preferred embodiment of the device 100 of the invention, which may be realized separately or together with one or two or several or all other features of the invention, said exposition tunnel 110 has a longitudinal axis, i. e. an axis identical to the axis of a cylinder shape, which is arranged horizontally, i. e. parallel to the ground on which the device 100 is positioned when in operation. Devices 100 comprising said exposition tunnel 110 having the axis horizontally arranged are usual exposition "beds" receiving the subject 200 in a recumbent position. This is evaluated by most users of the device 100 to be very convenient and relaxing. Moreover, a particularly even irradiation of actinic radiation can be provided in accordance with the present invention. An example of such a device 100 is shown in Figure 1. As seen exemplarily from Figure 1, the device 100 comprises a first part 180 and a second part 190 of a chassis, and the first (in operation of the device 100: the upper) part 180 can be pivoted away from the second (in operation: the lower) part 190 along an axis parallel to the longitudinal axis of the exposition tunnel, so as to allow entering into the device 100, i. e. onto the exposition bed within the exposition tunnel 110, in an easy and convenient manner.

In one alternative preferred embodiment of the device 100 of the invention, said exposition tunnel 110 has a longitudinal axis, i. e. an axis identical to the axis of a cylinder shape, which is arranged vertically, i. e. perpendicular to the ground on which the device 100 is positioned when in operation. Devices 100 comprising said exposition tunnel 110 having the axis vertically arranged are exposition "cabins" receiving the subject 200 in an erect or standing position. This is evaluated by most users of the device 100 to be very dynamic while in move. Moreover, an even irradiation of actinic radiation can be provided in accordance with the present invention in a device which is very space-saving. An example of such a device 100 is shown in Figure 2. As seen exemplarily from Figure 2, the device 100 comprises a first (or front) part 180 and a second (or rear) part 190 of a chassis, and the first (in operation of the device 100: the front) part 180 can be pivoted away, in a manner similar to a door, from the second (in operation: the rear) part 190 along an axis parallel to the longitudinal axis of the exposition tunnel, so as to allow entering into the device 100, i. e. into the exposition cabin within the exposition tunnel 110, in an easy and convenient manner.

Depending upon the type of device 100 (i. e. accommodating the subject in a recumbent or erect position), said exposition tunnel 110 is formed of one semi-cylinder barrel-shape first surface 120 and of at least one second surface 140, wherein said at least one second surface 140 is capable of complementing the first surface 120 in forming said exposition tunnel 110. This means that, in specific embodiments of the device 100 of the invention, an exposition tunnel 110 may be defined in shape and size by said first and second surfaces 120, 140 completely.

In more preferred embodiments of the device 100 of the invention, which may be realized separately or together with one or two or several or all other features of the invention, said exposition tunnel 110 is formed of said one semi-cylinder barrel-shape surface 120 and of at least one substantially flat surface 142. Preferably, this is an embodiment of the device 100 having the shape of an exposition "bed" on which the subject may be exposed to directed actinic radiation in a recumbent position: The subject 200 is positioned on said substantially flat surface 122 which, in this embodiment, is in a horizontal arrangement. In such an embodiment, said one semi-cylinder barrel-shape surface 120 is arranged above said substantially flat surface 142, usually capable of being moved away from said flat surface 142 by pivoting said semi-cylinder barrel-shape surface 120 around an axis parallel to one side edge of said flat surface 142 so as to "open" the exposition tunnel 110 for an easy entry of the subject 200 for actinic radiation exposure.

The term "substantially flat" in connection to the subject's lying surface 142, as used in the present specification and claims, is understood to mean a surface which extends into a horizontal plane substantially without recesses and elevations. As is usual, however, in the present field of the art, such a lying surface for the subject 100 to be exposed to actinic radiation may include recesses for a positioning of the subject's head, back, arms or legs, without substantially departing from the overall flatness.

In similarly preferred alternative embodiments of the device 100 of the invention, which may be realized separately or together with one or two or several or all other features of the invention, said exposition tunnel 110 is formed of said one semi-cylinder barrel-shape surface 120 and at least of one further, or second, semi-cylinder barrel-shape surface 141. Preferably, this is an embodiment of the device 100 having the shape of an exposition "cabin" within which the subject may be exposed to directed actinic radiation in an erect position: The subject 200 is positioned between said two semi-cylinder barrel-shape surfaces 120, 141 which, in this embodiment, are in a vertical arrangement. In such an embodiment, said one semi-cylinder barrel-shape surface 120 is arranged side by side with said second semi-cylinder barrel-shape surface 141 so as to form a full cylinder. One part of said cylinder usually is capable of being moved away from said second semi-cylinder barrel-shape surface 141 by pivoting said semi-cylinder barrel-shape surface 120 around an axis parallel to one vertical edge of said second semi-cylinder barrel-shape surface 141 so as to "open" the exposition tunnel 110 for an easy entry of the subject 200 for actinic radiation exposure. The subject 200 stands in the center of a cylinder-shape space formed by the two semi-cylinder barrel-shape surfaces 120, 141 and, while being exposed to actinic radiation, optionally, is turning around his/her own vertical axis.

In similarly preferred alternative embodiments of the device 100 of the invention, which may be realized separately or together with one or two or several or all other features of the invention, said exposition tunnel 110 comprises at least one further surface 143 which, even more preferably, is either arcuated or substantially flat. Such a preferred embodiment of the invention is exemplarily shown in Figure 1. In said embodiment shown in Figure 1, said additional surface 143 may serve forming a side wall of the exposition tunnel 110 to which the pivotable semi-cylinder barrel-shape surface 120 is connected. Such a further surface 143 of the device's exposition tunnel 110 creates a certain asymmetry of the exposition tunnel 110 for which the present invention is particularly useful.

In accordance with the invention, the semi-cylinder barrel-shape first surface 120, and the at least one second surface 140 as well, are made of a material substantially permeable to said actinic radiation to be applied to the subject 200. The term "material substantially permeable to said actinic radiation" as used in the present specification and claims, is understood to mean that the material of the surfaces is of such a nature that at least that portion of the actinic radiation emitted by the light sources for having the desired photochemical effect or photobiochemical effect can pass said walls without that substantial amounts thereof are absorbed by the surface material. A skilled person in this technical field knows such materials and may select them from a number of materials in accordance with the requirements of a specific case. In particularly preferred embodiments of the invention, the surfaces 120, 140 are made from a material selected from the group of actinic light-permeable polymeric materials, and acrylic polymers are particularly preferred, as also in the prior art. Also other known prior art materials capable of allowing a passage of substantially all actinic radiation directed thereto may be used alone or in combination.

In an even more preferred embodiment of the invention, said additional surface 143, similar to the semi-cylinder barrel-shape first surface 120 and the at least one second surface 140, is also made of a material substantially permeable to said actinic radiation to be applied to the subject 200.

In accordance with the invention, the device 100 for an application of directed actinic radiation to a subject 200 is shaped in a way that the surfaces 120, 140 and, optionally also any additional surface 143, of the exposition tunnel 110 separate an inner space 130, where a subject 200 to be exposed to actinic radiation is surrounded by said surfaces and is exposed to said actinic radiation, from outer spaces 150, 160 where light sources 170 capable of emitting actinic radiation through said surfaces 120, 140 are mounted in such a way that said light sources can emit said actinic radiation through said surfaces 120, 140 towards the subject 200. Such outer spaces 150, 160 may have any shape a skilled person may consider suitable for the purpose of, *inter alia,* accommodating said light sources 170, without that the invention is restricted in any way. Particularly, the shape of said outer spaces 150, 160 may depend on the type of device 100 used.

In preferred embodiments of the device 100 of the invention, which may be realized separately or together with one or two or several or all other features of the invention, if said exposition tunnel 110 has a longitudinal axis arranged horizontally, as shown in Figure 1, upper light sources 170u capable of emitting actinic radiation through said surface 120 downwards to the subject 200 lying in the recumbent position on said substantially flat surface 142, and lower light sources 1701 capable of emitting actinic radiation through said surface 140 upwards to the subject 200 lying in the recumbent position are mounted above said surface 120 in said outer space 150 and below said surface 140, 141, 142, in said outer space 160. In this embodiment, the outer spaces 150, 160 are upper and lower spaces and are used, *inter alia,* for an accommodation of said upper and lower light sources 170u, 1701. The outer spaces 150, 160 may be used in accordance with the invention for other purposes, too, which are described below in further detail.

In alternative preferred embodiments of the device 100 of the invention, which may be realized separately or together with one or two or several or all other features of the invention, if said exposition tunnel 110 has a longitudinal axis arranged vertically (as shown in Figure 2), front side light sources 170fs capable of emitting actinic radiation through said surface 120 backwards to the subject 200 standing in an erect position in said inner space 130 surrounded by said surfaces 120, 140, and back side light sources 170bs capable of emitting actinic radiation through said surface 140 forwards to the standing subject 200 are mounted on the outer sides of said surfaces 120, 140 in said outer spaces 150, 160. In this alternative embodiment, the outer spaces 150, 160 are front side outer spaces and back side outer spaces, which are used, *inter alia,* for an accommodation of said front side and back side light sources 170fs, 170bs. The outer spaces 150, 160 may be used in accordance with the invention for other purposes, too, which are described below in further detail.

In alternative preferred embodiments of the device 100 of the invention, which may be realized separately or together with one or two or several or all other features of the invention, if said exposition tunnel 110 comprises at least one additional arcuated or substantially flat surface 143 as described above, additional light sources 170a capable of emitting actinic radiation through said surface 143 onto the lying or standing subject 200 are mounted on the outer side of said surface 143 in said outer space 150 or on the outer side of said surface 143 in said outer spaces 150, 160. In such a case, for example, an application of directed actinic radiation to the subject 200 may be effected also from an additional side, e. g. in the case shown in Figure 1 from the right-hand side, in relation to the subject 200.

In accordance with the invention, said outer spaces 150, 160 are housed in at least first and second parts of a chassis 180, 190 of the device 100 and comprise means 181, 191 for fixedly holding, and electrically and electronically operating, said light sources 170 and/or means 182, 192 providing said at least first and second parts of the chassis 180, 190, and the components therein, for example said light sources 170 emitting actinic radiation to the subject 200 with electric power and/or means 183, 193 capable of ventilating, heating and/or cooling the device 100; and means 184, 194 for the further operation of the device 100, as for example control means and/or at least one processing unit for controlling the operation of the device 100 and/or for allowing external control means to be connected. Further means as known to a skilled person from similar devices of the prior art may also be mounted, singly or in combination, to the outer spaces 150, 160 of the first and second parts 180, 190 of the chassis, as for example handles for opening and closing the movable chassis parts 180, emergency switches capable of stopping the operation of the device 100 in cases of emergency, nozzles (and their operation channels and pumps) for allowing an access of fresh, cooling or warming air or liquid sprays to the exposition tunnel 130, a central processing unit capable of operating the device 100, actinic radiation sensors, a skin colour/skin status sensor for determining the subject's/user's skin condition as far as relevant for an actinic radiation exposure, to enumerate only few examples.

In preferred embodiments of the invention, which may be realized separately or together with one or two or several or all other features of the invention, the subject 200 is a human being 200 to be exposed to said directed actinic radiation. Human beings 200 to be exposed to said directed actinic radiation are sometimes also referred to, in this specification, as "users" of the device 100.

In accordance with the invention, the device 100 for an application of directed actinic radiation to a subject 200 further comprises at least one means 171 capable of controlling, and optionally varying, a radiant flux Φ of at least one actinic radiation-emitting light source 170 by changing the at least one light source's electronic parameters in accordance with the geometric position of said at least one actinic radiation-emitting light source 170, relative to its distance from the inner space 130, thereby achieving an even radiance E at the subject 200 exposed to said actinic radiation.

Means 171 capable of controlling the radiant flux Φ of the at least one actinic radiation-emitting light source 170 are, generally, well known to a skilled person in this technical field and may be selected from various such well-known means in accordance with the requirements of the specific case.

The term "radiant flux Φ", as used in the present specification and claims, is understood to relate to the radiant power or "radiation/light energy power" emitted by the respective radiation/light source. If a radiation/light source having a certain radiant flux Φ (more precisely: radiant flux Φₑ, measured in the SI unit: W) is emitting radiation/light onto a surface having the area A (measured in the SI unit: m²), such an area A is receiving radiation/light at a radiance E (more precisely: radiance Eₑ, measured in the derived SI unit W/m²), i. e. the "amount of radiation/light received per unit area" is Eₑ = dΦₑ/ dA [W/m²].

Assumed that any dependency of the radiation upon the radiation angle is not to be considered, the radiance Eₑ is dependent upon the distance (d, measured in the SI unit m) between the radiation/light source and the receiving surface area A in an inverse square proportion (1 / d² [m⁻²]).

The geometric relation between the actinic radiation-emitting radiation source 170 and its radiant flux Φₑ and the irradiance Eₑ received on the receiving surface A at a distance d (e. g. the subject/user 200 positioned in the inner space 130 of the device 100 of the invention and his/her skin surface) is shown in detail in Figure 4, generally. In a theoretical approach (also a part of irradiance measurements in international standards for determining the irradiance Eₑ in UV radiation-emitting apparatus), the subject/user 200 is assumed to be surrounded by a cylinder-shape shell 210 (in the case of an exposition "cabin": coaxial with the "cabin" cylinder) or a semicylinder-shape shell 210 (in the case of an exposition bed as shown in Figure 1 in dotted lines: having its axis coaxial with a center line of the second surface 142) having a radius ("r" in Figure 1 in a dotted line) of 30 cm. The surface of said (semi-) cylinder shell 210 is assumed to be the surface A (see Figure 4), from which the distance d to the actinic radiation-emitting radiation source 170 is determined, as a standard case for the evaluation of the geometric position of the subject/user 200 in the inner space 130 and his/her/its different distances to different radiation-emitting radiation sources 170 in the outer spaces 150, 160. As will be derived from Figure 4, the impingement point Pᵢ (where the actinic radiation is described above to impinge to the subject/user 200 is assumed to be located on said cylinder-shape (or semi-cylinder-shape) shell 210, in said theoretical approach.

It is shown Figure 4 exemplarily that, for two radiation sources 170, 170, which may have the same radiant flux Φₑ at their emission points Pₑ, the radiation may have to "travel" along different distances d₁ and d₂ before impinging onto the surface area A (wherein d₁ > d₂). As a result, the radiance Eₑ observed for the radiation source 170 closer to the surface A at the radiation impingement point Pᵢ on the surface A is different from (i. e. lager than) the radiance Eₑ observed for the radiation source farther away from the surface A at the radiation impinging point. As mentioned above, the surface A in Figure 4, with the impingement point Pᵢ, is considered, in said theoretical (standardized) approach, a part of the cylinder-shape (or semi-cylinder-shape) shell 210 surrounding the subject/user 200.

The term "geometric position of said at least one actinic radiation-emitting light source 170, relative to its distance from the inner space 130", is understood to have the following meaning, which is explained for one single radiation-emitting radiation source 170 (representing a plurality of radiation-emitting radiation sources) and a geometric receiving surface A (representing the (semi-)cylinder-shape shell 210 surrounding the subject/user 200 to be exposed to actinic radiation) (for the sake of simplicity) with reference to Figure 4:
As explained above, also with reference to Figures 1 and 2, the device 100 of the invention comprises the exposition tunnel 110 as the inner space 130, which is the space where the subject 200 is exposed to actinic radiation (and, theoretically, surrounded by said (semi-)cylinder-shape shell 210). Farther remote from said shell 210 (theoretically surrounding the subject 200), during an application of a directed actinic radiation, are the outer spaces 150, 160 where at least one light source 170, preferably two or three or several or a plurality of light sources 170, 170 capable of emitting actinic radiation are mounted. Hence, each point of the shell 210 in the inner space 130, where the subject is exposed to said actinic radiation emitted by said light source(s) 170, 170 has a distance, from said actinic radiation-emitting light source(s) 170, 170, which is different from the distance of any other point in said inner space 130, where the subject is exposed, too, and (theoretically) surrounded by said (semi-)cylinder-shape shell 210 to said actinic radiation emitted by said light source(s) 170, 170. Due to the fact that the effects of said actinic radiation, for the subject exposed to said radiation, are distance-dependent, as described above, the requirement of an even application of the directed actinic radiation to the subject 200 can be achieved only if the distance-dependent effects are equalized.

Such an equalization of the distance-dependent effects is achieved, in accordance with the invention, by controlling, and varying, the radiant flux Φ of at least one actinic radiation-emitting light source 170, preferably of a plurality of, or even more preferred of all, the light sources 170, 170 emitting actinic radiation from any of the outer spaces 150, 160 to the (theoretical) (semi-)cylinder-shape shell 210 surrounding the subject 200 positioned in said inner space 130.

Such a control, and variation, is exerted taking into account that the distance from each (for example first) light source 170 mounted in any of the outer spaces 150, 160, for example in the outer space 150, to the inner space 130, where the subject 200 (theoretically surrounded by the (semi-)cylinder-shape shell 210) is positioned, and any other distance from any other (for example second) light source 170 mounted in any of the outer spaces 150, 160, for example in the outer space 150, to the inner space 130 with the subject 200 positioned therein are different from each other.

The reason may be seen easily from Figures 1, 2, and 4 and is twofold:
The (semi-)cylinder-shape shell 210 (theoretically) surrounding the subject 200 positioned in said inner space is a regular geometrical "body" having round contours and forming surface areas which cannot be followed by the actinic radiation-emitting light sources' arrangement in said outer spaces 150, 160, on the one hand, thereby creating a certain subject geometry requirement.

On the other hand, the mountings of the actinic radiation-mitting light sources 170, 170 have to obey certain (sometimes standardized) device 100 requirements as, for example, the shape of the outer spaces 150, 160 or even the shape of the first and second parts of the device's chassis 180, 190, thereby creating a certain geometric device chassis requirement. In addition, any asymmetry of the device will have to be taken into account, as - for example - the asymmetry caused by the presence of the additional arcuated or substantially flat surface 143 (and its light sources) on the right-hand side of the device 100 shown in Figure 1.

Hence, in order to equalize the different distances the actinic radiation has to "travel" from the "emission point" Pₑ at the light source to the "impingement point" Pᵢ at the (semi-)cylinder-shape shell 210 surface, the radiant flux Φ of the actinic light-emitting light sources is carefully controlled and varied in accordance with said positional geometry requirements.

In the present invention, the term "actinic light", as defined above, comprises radiation in the broadest sense, but in any case excludes light which, even at lowest radiant powers, damages the subject to be irradiated. In preferred embodiments of the invention (i. e. in preferred embodiments of the device 100 of the invention and of the method of the invention), the term "actinic light" refers to light/radiation in the UV wavelength ranges, visible wavelength ranges and near IR wavelength ranges. In even more preferred embodiments of the device 100 and of the method of the invention, the term "actinic light" refers to radiation in the UV-B and UV-A wavelength ranges (280 to 315 nm and 315 to 400 nm), radiation in the visible wavelength range (400 to 800 nm) and radiation in the near IR wavelength range (800 to 950 nm, preferably 800 to 850 nm).

In the present invention, the electronic parameters of at least one light source 170 are changed electronically in accordance with the geometric position of said at least one actinic radiation-emitting light source 170. In more preferred embodiments of the invention, which may be realized separately or together with one or two or several or all other features of the invention, the electronic parameters of a plurality of light sources 170, 170 and even more preferred embodiments all light sources 170, 170 are changed electronically in accordance with the geometric position of the respective actinic radiation-emitting light sources 170, 170 relative to the inner space 130 of the device 100 with the subject 200 to be exposed positioned therein.

In even more preferred embodiments of the present invention, which may be realized separately or together with one or two or several or all other features of the invention, the electronic parameters of groups of light sources 170, 170 are changed electronically in accordance with the geometric position of the respective groups of actinic radiation-emitting light sources 170, 170 relative to the inner space 130 of the device 100 with the subject 200 to be exposed positioned therein.

In accordance with the invention, one means 171 capable of controlling a radiant flux Φ of at least one actinic radiation-emitting light source 170 may be used, or two or several or a plurality of such means 171, 171 capable of controlling a radiant flux Φ of at least one actinic radiation-emitting light source may be used. It is preferred in more preferred devices 100 of the invention that the number of means 171 capable of controlling a radiant flux Φ of at least one actinic radiation-emitting light source substantially corresponds to the number of actinic radiation-emitting light sources 170. In such a preferred embodiment, an excellent control of the radiation flux φ of said actinic radiation-emitting light sources 170 may be achieved.

In accordance with the invention, said means 171 capable of controlling, and optionally varying, a radiant flux Φ of at least one actinic radiation-emitting light source 170 comprise at least one, preferably more than one, electronic control member(s) 171, 171 capable of changing the light sources' electronic parameters in accordance with the geometric position of said actinic radiation-emitting light sources 170, relative to its distance from the inner space 130 and from the subject 200 positioned in said inner space 130 for receiving directed actinic radiation. Such more than one or plurality of electronic control member(s) 171 allow an exact distance-dependent control of the radiation flux Φ of said actinic radiation-emitting light sources 170.

In even more preferred embodiments of the invention, which may be realized separately or together with one or two or several or all other features of the invention, each actinic light-emitting light source 170, 170 is provided with at least one, preferably with one, electronic control member 171, 171 capable of changing the light sources' electronic parameters in accordance with the geometric position of said one actinic radiation-emitting light source 170, relative to its distance from the inner space 130 and from the (semi-)cylinder-shape shell 210 (theoretically) surrounding the subject 200 positioned in said inner space 130 for being applied with said directed actinic radiation.

In an alternative more preferred embodiment of the invention, which may be realized separately or together with one or two or several or all other features of the invention, a group of actinic light-emitting light sources 170, preferably a plurality of groups of actinic light-emitting light sources 170, 170, is provided with at least one, preferably with one or a plurality of, electronic control member(s) 171, 171 capable of changing the light sources' electronic parameters in accordance with the geometric position of said one actinic radiation-emitting light source 170, relative to its distance from the inner space 130 and from the subject 200 positioned in said inner space 130 for being applied with said directed actinic radiation.

What concerns the light sources' electronic parameter(s) to be controlled and varied, a change of one electronic parameter may occur, or a change of two or of several electronic parameters may occur. It is even possible that, for one actinic light-emitting light source 170, one electronic parameter X may be controlled and optionally varied and, for another actinic light-emitting light source 170, another electronic parameter Y may be controlled and optionally varied (X and Y refer to two different electronic parameters of actinic light-emitting light sources 170, 170, particularly of two different electronic parameters of different actinic light-emitting light sources 170, 170). A person skilled in this technical field known these electronic parameters and the possibility of changing such electronic parameters, and may select those electronic parameters in accordance with the requirements of a specific case, e. g. of a specific device 100 of the invention or of a specific method of the invention. Hence, the invention does not impose any restriction to such an electronic parameter selection.

In further preferred embodiments of the invention, which may be realized separately or together with one or two or several or all other features of the invention, the light source(s)' electronic parameter changed by said at least one electronic control member 171 is selected from the group consisting of the light source's power, voltage, current, frequency, and combinations thereof.

In particularly preferred embodiments of the invention, which may be realized separately or together with one or two or several or all other features of the invention, the change of the light source(s)' electronic parameter(s) is a change resulting from: a determination of the geometric position of said at least one radiation-emitting radiation source 170 and of its distance d from a selected position within the inner space 130 of the device 100; a determination of the required change of the radiant flux Φ of said at least one radiation source for achieving a required even radiance E at said distance d; a generation of one or more than one signal(s), by a processing unit of the device 100, capable of effecting a change of the light source(s)' electronic parameter(s) in dependency upon the light source(s)' distance d from the inner space 130; a transmission of said signal(s) from the processing unit of the device 100 to at least one electronic control member 171 capable of varying the radiant flux Φ of said at least one radiation-emitting radiation source 170; wherein said at least one control member 171 effects a variation of the radiant flux Φ of said at least one radiation-emitting radiation source 170, by changing at least one selected from the light source's power, voltage, current, frequency, and combinations thereof, in coordination, by the processing unit of the device 100, with effecting variations of the radiant flux Φ of at least one further radiation-emitting radiation source 170, preferably of several other, or a group of other, radiation-emitting light sources 170, 170, dependent upon the source(s)' distances d, d, from a respective selected position within the inner space 130 of the device 100.

The above-described device 100 was found to be suitable for achieving all the above objects of the invention. In particular, the radiant intensity of all actinic radiation-emitting light sources 170 could be controlled reliably, and varied effectively, in such a manner that an even irradiance E can be achieved over the whole body surface of the user. Thereby, the efficiency of irradiation devices 100 and of the irradiation method could be enhanced with the present invention, and over-exposures on the subject's side could be prevented in the same way as under-exposures, i. e. an insufficient result of the application of a directed actinic radiation.

The invention also relates to a non-therapeutical method for operating a device 100, said device 100 applying a directed actinic radiation to a subject 200.

In accordance with the present invention, said non-therapeutical method comprises the step of providing a device 100 as described above in all details in the broadest sense and in its preferred embodiments; and the step of operating at least one of the device's actinic light radiation-emitting light sources 170, 170 by controlling, and optionally varying, at least a radiant flux Φ of at least one of said actinic radiation-emitting light sources 170 by changing at least one of its electronic parameters in accordance with the geometric position of said at least one actinic radiation-emitting light source 170, relative to its distance from the inner space 130 of the device 100, thereby achieving an even radiance E at exposure to said actinic radiation emitted by said light source 170.

The device 100 provided for conducting the non-therapeutical method of the invention was described above in all details. Hence, there is no need for another description of said device 100, here, and reference is made, in all aspects of the apparatus to be provided in the present non-therapeutical method, to the above detailed description of the device 100.

In accordance with the non-therapeutical method of the present invention, in the device 100 described above, at least one of the device's actinic light radiation-emitting light sources 170, 170 is operated by controlling, and optionally varying, at least a radiant flux Φ of at least one of said actinic radiation-emitting light sources 170 by changing at least one of its electronic parameters in accordance with the geometric position of said at least one actinic radiation-emitting light source 170, relative to its distance from the inner space 130 of the device 100, particularly from the (semi-)cylinder-shape shell 210, relative to the inner space 130 of the device 100 with the subject 200 to be exposed positioned therein, thereby achieving an even radiance E at exposure to said actinic radiation emitted by said light source 170.

In accordance with the non-therapeutical method of the invention, the number of the device's actinic light-emitting light sources to be specifically operated is not restricted, and a skilled person knows, or may easily find out by simple tests, how many of said light sources 170 of the device may be operated in a specific manner of the invention.

In preferred embodiments of the method of the invention, which may be realized separately or together with one or two or several or all other features of the invention, the non-therapeutical method of the invention comprises operating single ones of, or groups of, or all of, the device's actinic light radiation-emitting light sources 170, 170, by controlling at least a radiant flux Φ of said actinic radiation-emitting light source(s) 170, 170 by changing at least one of its/their electronic parameter(s) in accordance with the geometric position of said actinic radiation-emitting light source(s) 170, 170, relative to its/their distance from the inner space 130 of the device 100, particularly from the (semi-) cylinder-shape shell 210, thereby achieving an even radiance E at exposure to said actinic radiation emitted by said light source(s) (170, 170).

What concerns the light sources' electronic parameter to be controlled and varied by the method of the present invention, one electronic parameter may be changed, or two or several electronic parameters may be changed. It is even possible that, for one actinic light-emitting light source 170, one electronic parameter X may be controlled and optionally varied/changed and, for another actinic light-emitting light source 170, another electronic parameter Y may be controlled and optionally varied/changed (X and Y refer to two different electronic parameters of different actinic light-emitting light sources 170, 170, examples thereof being given below). A person skilled in this technical field knows these electronic parameters and the possibility of changing such electronic parameters, and may select those electronic parameters in accordance with the requirements of a specific case, e. g. of a specific non-therapeutical method of the invention. Hence, the invention does not impose any restriction to such an electronic parameter selection.

In preferred embodiments of the non-therapeutical method of the invention, which may be realized separately or together with one or two or several or all other features of the invention, the method of the invention comprises the step that said at least one electronic parameter is controlled and optionally varied/changed by using at least one electronic control member 171. Such electronic control members 171 for actinic light-emitting light sources 170 may be used singly or in combination for two or several or a plurality of actinic light-emitting light sources 170, 170. Alternatively, such electronic control members 171 may be used to control, and optionally vary, electronic parameters of single or a plurality of group(s) of actinic light-emitting light sources 170, 170.

In further preferred embodiments of the method of the invention, which may be realized separately or together with one or two or several or all other features of the invention, the non-therapeutical method of the invention comprises a step wherein the light source(s)' electronic parameter selected from the group consisting of the light source's power, voltage, current, frequency, and combinations thereof is changed by said at least one electronic control member 171.

In further preferred embodiments of the method of the invention, which may be realized separately or together with one or two or several or all other features of the invention, the non-therapeutical method of the invention comprises the steps of changing the light source(s)' electronic parameter(s) by determining the geometric position of said at least one radiation-emitting radiation source 170 and of its distance d from a selected position within the inner space 130 of the device 100; determining the required change of the radiant flux Φ of said at least one radiation source for achieving a required even radiance E at said distance d; generating one or more than one signal(s), by a processing unit of the device 100, capable of effecting a change of the light source(s)' electronic parameter(s) in dependency upon the light source(s)' distance d from the inner space 130; transmitting said signal(s) from the processing unit of the device 100 to at least one electronic control member 171 capable of varying the radiant flux Φ of said at least one radiation-emitting radiation source 170; thereby causing said at least one control member 171 to effect a variation of the radiant flux Φ of said at least one radiation-emitting radiation source 170, by changing at least one selected from the light source's power, voltage, current, frequency, and combinations thereof, in coordination, by the processing unit of the device 100, with effecting variations of the radiant flux Φ of at least one further radiation-emitting radiation source 170, preferably of several other, or a group of other, radiation-emitting light sources 170, 170 dependent upon the source(s)' distances d, d, from a respective selected position within the inner space 130 of the device 100.

In preferred embodiments of the method of the invention, which may be realized separately or together with one or two or several or all other features of the invention, the non-therapeutical method of the invention comprises the step that the determination and electronic property variation steps are conducted at a plurality of the device's radiation-emitting radiation sources 170, 170, or at groups of the device's radiation-emitting radiation sources 170, 170, in dependency upon the light sources' geometric position, i. e. distance d from a respective selected position within the inner space 130 of the device 100. Thereby, an even radiance E can be achieved in the inner space 130 and at the surface of the (theoretical) (semi-)cylinder-shape shell 210 surrounding the subject/user 200 exposed to said actinic radiation in said inner space 130.

Particularly (but without restriction), the irradiating device 100 of the present invention having high evenness of irradiation, and the non-therapeutical even irradiation method of the invention as well, may be used for tanning the human body by the activation of melanin generation and melanin conversion into the dark (brown) form of the pigment naturally occurring in the human skin through an application, to the skin of a subject, e. g. to the skin of a human being, of actinic radiation having a wavelength λ in the range of from 280 to 315 nm (UV-B radiation) and/or of actinic radiation having a wavelength λ in the range of from 315 to 400 nm (UV-A radiation) for serving purposes in the cosmetic and/or medical field(s).

In another embodiment of the invention, the irradiating device 100 of the present invention having high evenness of irradiation, and the non-therapeutical even irradiation method of the invention as well, may be used for applying short wavelength UV-B radiation in the wavelength range of λ = 297 nm ± 10 nm with the aim of promoting Vitamin D biosynthesis from Vitamin D precursors in the human skin. Moreover, in a further embodiment of the invention, the irradiating device 100 of the present invention having high evenness of irradiation, and the non-therapeutical even irradiation method of the invention as well, may be used for applying actinic radiation having wavelengths λ in the visible and near IR (infrared) range (400 or, even better, > 550 nm to 850 nm), to the human skin with the aim of activating the biosynthesis of beneficial compounds for the skin's nourishing and rejuvenation, as for example elastin, keratin and hyaluronic acid.

In the above description, the invention was described by referring to the Figures and to the description of preferred embodiments. The invention is, however, not restricted to the embodiments or to the uses shown in the Figures and/or described in the specification. These embodiments shown and/or described are only exemplary for the invention and serve an explanation of the invention and a better and complete understanding thereof.

### Reference Numerals

- 100: Device for an application of directed actinic radiation
- 110: Exposition tunnel
- 120: Semi-cylinder barrel-shape first surface
- 130: Inner space
- 140: Second surface
- 141: Semi-cylinder barrel-shape second surface
- 142: Substantially flat (second) surface
- 143: Additional arcuated or substantially flat surface
- 150, 160: Outer spaces
- 170: Light source(s)
- 170u/170l: Upper light sources/lower light sources
- 170fs/170bs: Front side/back side light sources
- 170a: Additional light sources
- 171: Means capable of controlling radiant flux Φ of light sources
- 180, 190: First/second parts of the chassis
- 181, 191: Means for holding and operating light sources 170
- 182, 192: Means providing first/second chassis parts 180,190 with power
- 183, 193: Heating/cooling means
- 184, 194: Device operation means, including a processing unit
- 200: subject/user
- 210: (semi-)cylinder-shape shell

- r: Radius of the(semi-)cylinder-shape shell
- Pₑ: Emission point of actinic radiation at a radiation source 170
- Pᵢ: Impingement point of actinic radiation at surface A or shell 210
- d: Distance between Pₑ and Pᵢ
- A: Surface receiving actinic radiation

## Claims

1. A device (100) for an application of directed actinic radiation to a subject (200), said device (100) comprising:
- an exposition tunnel (110) being capable of surrounding said subject (200) to be exposed to said actinic radiation, said exposition tunnel (110) being formed of one semi-cylinder barrel-shape first surface (120) made of a material substantially permeable to said actinic radiation to be applied to the subject (200), and of at least one second surface (140) made of said material substantially permeable to said actinic radiation to be applied to the subject (200), said at least one second surface (140) being capable of complementing the first surface (120) in forming said exposition tunnel (110);
- the surfaces (120, 140) of said exposition tunnel (110) separating an inner space (130) where a subject (200) is exposed to said actinic radiation, from outer spaces (150, 160) where light sources (170) capable of emitting actinic radiation through said surfaces (120, 140) are mounted;
- said outer spaces (150, 160) being housed in at least first and second parts of a chassis (180, 190) of the device (100) and comprise means for fixedly holding, and electrically and electronically operating, said light sources (170); means (182, 192) providing said at least first and second parts of the chassis (180, 190) with electric power; means capable of ventilating, heating and/or cooling the device (100); and means (184, 194) for the operation of the device (100), including a processing unit;
said device (100) further comprising
- at least one means (171) capable of controlling, and optionally varying, a radiant flux Φ of at least one actinic radiation-emitting light source (170) by changing the at least one light source's electronic parameters in accordance with the geometric position of said at least one actinic radiation-emitting light source (170), relative to its distance from the inner space (130), thereby achieving an even radiance E at the subject (200) exposed to said actinic radiation,
wherein said means (171) capable of controlling, and optionally varying, a radiant flux Φ of at least one actinic radiation-emitting light source (170) comprise at least one or more than one electronic control member (171) changing the light sources' electronic parameters in accordance with the geometric position of said at least one actinic radiation-emitting light source (170), relative to its distance from the inner space (130).

2. The device (100) as claimed in claim 1, the device being configured to irradiate a human being to be exposed to said directed actinic radiation.

3. The device (100) as claimed in claim 1 or claim 2, wherein said exposition tunnel (110) has a longitudinal axis arranged horizontally or wherein said exposition tunnel (110) has a longitudinal axis arranged vertically.

4. The device (100) as claimed in any one of the claims 1 to 3, wherein said exposition tunnel (110) is formed of said one semi-cylinder barrel-shape surface (120) and of a at least one substantially flat surface (122), or wherein said exposition tunnel (110) is formed of said one semi-cylinder barrel-shape surface (120) and of a second semi-cylinder barrel-shape surface (121).

5. The device (100) as claimed in claim 4, wherein said exposition tunnel (110) comprises at least one additional arcuated or substantially flat surface (143), which preferably is also made of a material substantially permeable to said actinic radiation to be applied to the subject (200).

6. The device (100) as claimed in any one of the claims 1 to 5, wherein, if said exposition tunnel (110) has a longitudinal axis arranged horizontally, upper light sources (170u) capable of emitting actinic radiation through said surface (120) downwards to the lying subject (200) and lower light sources (170l) capable of emitting actinic radiation through said surface (140) upwards to the lying subject (200) are mounted above said surface (120) in said outer space (150) and below said surface (140) in said outer space (160); or, if said exposition tunnel (110) has a longitudinal axis arranged vertically, front side light sources (170fs) capable of emitting actinic radiation through said surface (120) backwards to the standing subject (200) and back side light sources (170bs) capable of emitting actinic radiation through said surface (140) forwards to the standing subject (200) are mounted on the outer sides of said surfaces (120, 140) in said outer spaces (150, 160).

7. The device (100) as claimed in any one of the claims 1 to 6, wherein, in the case that said exposition tunnel (110) comprises at least one additional arcuated or substantially flat surface (143), additional light sources (170a) capable of emitting actinic radiation through said surface (143) onto the lying or standing subject (200) are mounted on the outer side of said surface (143) in said outer space(s) (150, 160).

8. The device (100) as claimed in any one of the claims 1 to 7, wherein each actinic light-emitting light source (170) is provided with at least one, preferably with one, electronic control member (171) changing the light sources' electronic parameters in accordance with the geometric position of said one actinic radiation-emitting light source (170), relative to its distance from the inner space (130) and from the (theoretical) (semi-) cylinder-shape shell (210) surrounding the subject (200) positioned in said inner space (130) for being applied with directed actinic radiation.

9. The device (100) as claimed in any one of the claims 1 to 7, wherein a group of actinic light-emitting light sources (170), preferably a plurality of groups of actinic light-emitting light sources (170, 170), is provided with at least one, preferably with one or a plurality of, electronic control member(s) (171, 171) changing the light sources' group's electronic parameters in accordance with the geometric position of said group of at least one actinic radiation-emitting light source (170), relative to its distance from the inner space (130).

10. The device (100) as claimed in any one of the claims 1 to 9, wherein the light source(s)' electronic parameter changed by said at least one electronic control member is selected from the group consisting of the light source's power, voltage, current, frequency, and combinations thereof.

11. The device (100) as claimed in any one of the claims 1 to 10, wherein the change of the light source(s)' electronic parameter(s) is a change resulting from: a determination of the geometric position of said at least one radiation-emitting radiation source (170) and of its distance d from a selected position within the inner space (130) of the device (100); a determination of the required change of the radiant flux Φ of said at least one radiation source for achieving a required even radiance E at said distance d; a generation of one or more than one signal(s), by a processing unit of the device (100), capable of effecting a change of the light source(s)' electronic parameter(s) in dependency upon the light source(s)' distance d from the inner space (130); a transmission of said signal(s) from the processing unit of the device (100) to at least one electronic control member (171) capable of varying the radiant flux Φ of said at least one radiation-emitting radiation source (170); wherein said at least one control member (171) effects a variation of the radiant flux Φ of said at least one radiation-emitting radiation source (170), by changing at least one selected from the light source's power, voltage, current, frequency, and combinations thereof, in coordination, by the processing unit of the device (100), with effecting variations of the radiant flux Φ of at least one further radiation-emitting radiation source (170), preferably of several other, or a group of other, radiation-emitting light sources (170, 170), dependent upon the source(s)' distances d, d, from a respective selected position within the inner space (130) of the device (100).

12. A non-therapeutical method for operating a device (100) applying a directed actinic radiation to a subject (200), said method comprising the steps of
- providing a device (100) as claimed in any one of the claims 1 to 11; and
- operating at least one of the device's actinic light radiation-emitting light sources (170) by controlling at least a radiant flux Φ of at least one of said actinic radiation-emitting light sources (170) by changing at least one of its electronic parameters in accordance with the geometric position of said at least one actinic radiation-emitting light source (170), relative to its distance from the inner space (130) of the device (100), thereby achieving an even radiance E at exposure to said actinic radiation emitted by said light source (170).

13. The method as claimed in claim 12, comprising operating single ones of, or groups of, or all of, the device's actinic light radiation-emitting light sources (170, 170) by controlling at least a radiant flux Φ of said actinic radiation-emitting light source(s) (170, 170) by changing at least one of its/their electronic parameter(s) in accordance with the geometric position of said actinic radiation-emitting light source(s) (170, 170), relative to its/their distance from the inner space (130) of the device (100) by means of at least one control member (171), thereby achieving an even radiance E at exposure to said actinic radiation emitted by said light source(s) (170, 170).

14. The method as claimed in claim 12 or claim 13, wherein the light source(s)' electronic parameter selected from the group consisting of the light source's power, voltage, current, frequency, and combinations thereof is changed by said at least one electronic control member (171).

15. The method as claimed in any one of the claims 12 to 14, said process comprising the steps of changing the light source(s)' electronic parameter(s) by determining the geometric position of said at least one radiation-emitting radiation source (170) and of its distance d from a selected position within the inner space (130) of the device (100); determining the required change of the radiant flux Φ of said at least one radiation source for achieving a required even radiance E at said distance d; generating one or more than one signal(s), by a processing unit of the device (100), capable of effecting a change of the light source(s)' electronic parameter(s) in dependency upon the light source(s)' distance d from the inner space (130); transmitting said signal(s) from the processing unit of the device (100) to at least one electronic control member (171) capable of varying the radiant flux Φ of said at least one radiation-emitting radiation source (170); thereby causing said at least one control member (171) to effect a variation of the radiant flux Φ of said at least one radiation-emitting radiation source (170), by changing at least one selected from the light source's power, voltage, current, frequency, and combinations thereof, in coordination, by the processing unit of the device (100), with effecting variations of the radiant flux Φ of at least one further radiation-emitting radiation source (170), preferably of several other, or a group of other, radiation-emitting light sources (170, 170), dependent upon the source(s)' distances d, d, from a respective selected position within the inner space (130) of the device (100).

## Patentansprüche

1. Vorrichtung (100) für eine Aufbringung von gerichteter aktinischer Strahlung auf eine Person (200), wobei die Vorrichtung (100) umfasst:
- einen Belichtungstunnel (110), der befähigt ist, die mit der aktinischen Strahlung zu bestrahlende Person (200) zu umgeben, wobei der Belichtungstunnel (110) gebildet ist aus einer ersten Fläche (120) in Form eines Halbzylinder-Fasses, hergestellt aus einem Material, das für die aktinische Strahlung, die auf die Person (200) aufgebracht werden soll, im Wesentlichen durchlässig ist, und aus wenigstens einer zweiten Fläche (140), die aus dem Material hergestellt ist, das für die aktinische Strahlung, die auf die Person (200) aufgebracht werden soll, im Wesentlichen durchlässig ist, wobei die wenigstens eine zweite Fläche (140) befähigt ist, die erste Fläche (120) darin zu ergänzen, den Belichtungstunnel (110) zu bilden;
- wobei die Flächen (120, 140) des Belichtungstunnels (110) einen Innenraum (130), wo eine Person (200) der aktinischen Strahlung ausgesetzt ist, von Außenräumen (150, 160) trennen, wo Lichtquellen (170) montiert sind, die befähigt sind, aktinische Strahlung durch die Flächen (120, 140) zu emittieren;
- wobei die Außenräume (150, 160) in wenigstens ersten und zweiten Teilen eines Gehäuses (180, 190) der Vorrichtung (100) untergebracht sind und umfassen: Einrichtungen zum festen Halten und elektrischen und elektronischen Betreiben der Lichtquellen (170); Einrichtungen (182, 192), die die wenigstens ersten und zweiten Teile des Gehäuses (180, 190) mit elektrischer Energie versorgen; Einrichtungen, die befähigt sind, die Vorrichtung (100) zu belüften, zu heizen und/oder zu kühlen; und Einrichtungen, (184, 194) für das Betreiben der Vorrichtung (100), einschließlich einer Prozessor-Einheit;
wobei die Vorrichtung (100) weiter umfasst:
- wenigstens eine Einrichtung (171), die befähigt ist, eine Strahlungsleistung Φ wenigstens einer aktinische Strahlung emittierenden Lichtquelle (170) dadurch zu steuern und gegebenenfalls zu ändern, dass sie die elektronischen Parameter der wenigstens einen Lichtquelle in Übereinstimmung mit der geometrischen Position der wenigstens einen aktinische Strahlung emittierenden Lichtquelle (170) relativ zu ihrer Entfernung von dem Innenraum (130) ändert, und dadurch eine gleichmäßige Bestrahlungsstärke E an der Person (200) erreicht, die der aktinischen Strahlung ausgesetzt ist;
- wobei die Einrichtungen (171), die befähigt sind, eine Strahlungsleistung Φ wenigstens einer aktinische Strahlung emittierenden Lichtquelle (170) zu steuern und gegebenenfalls zu ändern, wenigstens ein oder mehr als ein elektronisches Steuer-Element (171) umfassen, das die elektronischen Parameter der Lichtquellen in Übereinstimmung mit der geometrischen Position der wenigstens einen, aktinische Strahlung emittierenden Lichtquelle (170) relativ zu ihrer Entfernung von dem Innenraum (130) ändert.

2. Vorrichtung (100) wie in Anspruch 1 beansprucht, wobei die Vorrichtung so konfiguriert ist, dass sie ein menschliches Wesen bestrahlt, das der gerichteten aktinischen Strahlung ausgesetzt werden soll.

3. Vorrichtung (100) wie in Anspruch 1 oder Anspruch 2 beansprucht, wobei der Belichtungstunnel (110) eine Längsachse aufweist, die horizontal angeordnet ist, oder worin der Belichtungstunnel (110) eine Längsachse aufweist, die vertikal angeordnet ist.

4. Vorrichtung (100) wie in irgendeinem der Ansprüche 1 bis 3 beansprucht, wobei der Belichtungstunnel (110) gebildet ist aus der einen Fläche (120) in Form eines Halbzylinder-Fasses und aus wenigstens einer im Wesentlichen flachen Fläche (122), oder wobei der Belichtungstunnel (110) gebildet ist aus der einen Fläche (120) in Form eines Halbzylinder-Fasses und aus einer zweiten Fläche (121) in Form eines Halbzylinder-Fasses.

5. Vorrichtung (100) wie in Anspruch 4 beansprucht, wobei der Belichtungstunnel (110) wenigstens eine zusätzliche gebogene oder im Wesentlichen flache Fläche (143) umfasst, die vorzugsweise auch aus einem Material hergestellt ist, das im Wesentlichen durchlässig für die aktinische Strahlung ist, die auf die Person (200) aufgebracht werden soll.

6. Vorrichtung (100) wie in irgendeinem der Ansprüche 1 bis 5 beansprucht, wobei dann, wenn der Belichtungstunnel (110) eine Längsachse aufweist, die horizontal angeordnet ist, obere Lichtquellen (170u), die befähigt sind, aktinische Strahlung durch die Fläche (120) nach unten auf die liegende Person (200) zu emittieren, und untere Lichtquellen (170l), die befähigt sind, aktinische Strahlung durch die Fläche (140) nach oben auf die liegende Person (200) zu emittieren, oberhalb der Fläche (120) in dem Außenraum (150) und unterhalb der Fläche (140) in dem Außenraum (160) montiert sind; oder dann, wenn der Belichtungstunnel (110) eine Längsachse aufweist, die vertikal angeordnet ist, Vorderseiten-Lichtquellen (170fs), die befähigt sind, aktinische Strahlung durch die Fläche (120) nach hinten auf die stehende Person (200) zu emittieren, und Rückseiten-Lichtquellen (170bs), die befähigt sind, aktinische Strahlung durch die Fläche (140) nach vorn auf die stehende Person (200) zu emittieren, auf den Außenseiten der Flächen (120, 140) in den Außenräumen (150, 160) montiert sind.

7. Vorrichtung wie in irgendeinem der Ansprüche 1 bis 6 beansprucht, wobei in dem Fall, in dem der Belichtungstunnel (110) wenigstens eine zusätzliche gebogene oder im Wesentlichen flache Fläche (143) umfasst, zusätzliche Lichtquellen (170a), die befähigt sind, aktinische Strahlung durch die Fläche (143) auf die liegende oder stehende Person (200) zu emittieren, auf der Außenseite der Fläche (143) in dem Außenraum/den Außenräumen (150, 160) montiert sind.

8. Vorrichtung (100) wie in irgendeinem der Ansprüche 1 bis 7 beansprucht, wobei jede aktinisches Licht emittierende Lichtquelle (170) mit wenigstens einem, vorzugsweise mit einem, elektronischen Steuer-Element (171) versehen ist, das die elektronischen Parameter der Lichtquellen in Übereinstimmung mit der geometrischen Position der einen, aktinisches Licht emittierenden Lichtquelle (170) relativ zu ihrer Entfernung von dem Innenraum (130) und von der (theoretischen) (halb-) zylinderförmigen Schale (210) ändert, die die Person (200) umgibt, die in dem Innenraum (130) positioniert ist, um mit gerichteter aktinischer Strahlung beaufschlagt zu werden.

9. Vorrichtung (100) wie in irgendeinem der Ansprüche 1 bis 7 beansprucht, wobei eine Gruppe der aktinisches Licht emittierenden Lichtquellen (170), vorzugsweise eine Mehrzahl von Gruppen aktinisches Licht emittierender Lichtquellen (170, 170), versehen ist mit wenigstens einem, vorzugsweise mit einem oder einer Mehrzahl von, elektronischen Steuer-Element(en) (171, 171), das/die die elektronischen Parameter der Gruppen von Lichtquellen in Übereinstimmung mit der geometrischen Position der Gruppe von wenigstens einer aktinisches Licht emittierenden Lichtquelle (170) relativ zu ihrer Entfernung von dem Innenraum (130) ändert/ändern.

10. Vorrichtung (100) wie in irgendeinem der Ansprüche 1 bis 9 beansprucht, wobei der elektronische Parameter der Lichtquelle(n), der durch das wenigstens eine Steuer-Element geändert wird, gewählt ist aus der Gruppe, die besteht aus der Energie, der Spannung, dem Strom, der Frequenz der Lichtquelle und Kombinationen daraus.

11. Vorrichtung (100) wie in irgendeinem der Ansprüche 1 bis 10 beansprucht, wobei die Änderung des/der elektronischen Parameter(s) der Lichtquelle(n) eine Änderung ist, die resultiert aus: einer Bestimmung der geometrischen Position der wenigstens einen Strahlung emittierenden Strahlungsquelle (170) und ihrer Entfernung d von einer ausgewählten Position innerhalb des Innenraums (130) der Vorrichtung (100); einer Bestimmung der erforderlichen Änderung der Strahlungsleistung Φ der wenigstens einen Strahlungsquelle zum Erreichen einer erforderlichen gleichmäßigen Bestrahlungsstärke E bei der Entfernung d; einer Erzeugung eines Signals oder mehr als eines Signals durch eine Prozessor-Einheit der Vorrichtung (100), das befähigt ist, eine Änderung des/der elektronischen Parameter(s) der Lichtquelle(n) in Abhängigkeit von der Entfernung d der Lichtquelle(n) von dem Innenraum (130) zu bewirken; einer Übertragung des/der Signale(s) von der Prozessor-Einheit der Vorrichtung (100) an wenigstens ein elektronisches Steuer-Element (171), das befähigt ist, die Strahlungsleistung Φ der wenigstens einen Strahlung emittierenden Strahlungsquelle (170) zu ändern; wobei das wenigstens eine Steuer-Element (171) eine Änderung der Strahlungsleistung Φ der wenigstens einen Strahlung emittierenden Strahlungsquelle (170) bewirkt durch Änderung wenigstens eines Parameters gewählt aus der Gruppe Energie, Spannung, Strom, Frequenz der Lichtquelle und Kombinationen daraus, in Koordination mit einem Bewirken von Änderungen der Strahlungsleistung Φ wenigstens einer weiteren Strahlung emittierenden Strahlungsquelle (170), vorzugsweise von einigen anderen oder einer Gruppe von anderen Strahlung emittierenden Lichtquellen (170, 170) durch die Prozessor-Einheit der Vorrichtung (100), in Abhängigkeit von den Entfernungen d, d der Quelle(n) von einer jeweiligen ausgewählten Position innerhalb des Innenraums (130) der Vorrichtung (100).

12. Nicht-therapeutisches Verfahren zum Betreiben einer Vorrichtung (100), die eine gerichtete aktinische Strahlung auf eine Person (200) aufbringt, wobei das Verfahren die Schritte umfasst, dass man
- eine Vorrichtung (100) bereitstellt, wie sie in irgendeinem der Ansprüche 1 bis 11 beansprucht ist; und
- wenigstens eine der aktinische Lichtstrahlung emittierenden Lichtquellen (170) der Vorrichtung betreibt, indem man wenigstens eine Strahlungsleistung Φ wenigstens einer der aktinische Strahlung emittierenden Lichtquellen (170) dadurch steuert, dass man wenigstens einen ihrer elektronischen Parameter in Übereinstimmung mit der geometrischen Position der wenigstens einen aktinische Strahlung emittierenden Lichtquelle (170), bezogen auf ihre Entfernung von dem Innenraum (130) der Vorrichtung (100), ändert und dadurch eine gleichmäßige Bestrahlungsstärke E bei Bestrahlen durch die aktinische Strahlung erreicht, die durch die Lichtquelle (170) emittiert wird.

13. Verfahren wie in Anspruch 12 beansprucht, umfassend ein Betreiben von einzelnen der, oder von Gruppen der, oder von allen der aktinische Lichtstrahlung emittierenden Lichtquellen (170, 170) der Vorrichtung durch Steuern wenigstens einer Strahlungsleistung Φ der aktinische Strahlung emittierenden Lichtquelle(n) (170, 170) durch Ändern wenigstens eines ihrer/ihrer elektronischen Parameter in Übereinstimmung mit der geometrischen Position der aktinische Strahlung emittierenden Lichtquelle(n) (170, 170), bezogen auf ihre/ihre Entfernung von dem Innenraum (130) der Vorrichtung (100) mittels wenigstens eines Steuer-Elements (171), so dass dadurch eine gleichmäßige Bestrahlungsstärke E bei Bestrahlen mit der wenigstens einen Strahlung erreicht wird, die von der/den Lichtquelle(n) (170, 170) emittiert wird.

14. Verfahren wie in Anspruch 12 oder Anspruch 13 beansprucht, wobei der elektronische Parameter der Lichtquelle(n), der gewählt ist aus der Gruppe, die besteht aus Energie, Spannung, Strom, Frequenz der Lichtquelle und Kombinationen daraus, durch das wenigstens eine Steuer-Element (171) geändert wird.

15. Verfahren wie in irgendeinem der Ansprüche 12 bis 14 beansprucht, wobei das Verfahren die Schritte umfasst, dass man den/die elektronischen Parameter der Lichtquelle(n) ändert durch: ein Bestimmen der geometrischen Position der wenigstens einen Strahlung emittierenden Strahlungsquelle (170) und ihrer Entfernung d von einer ausgewählten Position innerhalb des Innenraums (130) der Vorrichtung (100); ein Bestimmen der erforderlichen Änderung der Strahlungsleistung Φ der wenigstens einen Strahlungsquelle zum Erreichen einer erforderlichen gleichmäßigen Bestrahlungsstärke E bei der Entfernung d; ein Erzeugen eines Signals oder mehr als eines Signals durch eine Prozessor-Einheit der Vorrichtung (100), das befähigt ist, eine Änderung des/der elektronischen Parameter(s) der Lichtquelle(n) in Abhängigkeit von der Entfernung d der Lichtquelle(n) von dem Innenraum (130) zu bewirken; ein Übertragen des/der Signale(s) von der Prozessor-Einheit der Vorrichtung (100) an wenigstens ein elektronisches Steuer-Element (171), das befähigt ist, die Strahlungsleistung Φ der wenigstens einen Strahlung emittierenden Strahlungsquelle (170) zu ändern; wobei bewirkt wird, dass das wenigstens eine Steuer-Element (171) eine Änderung der Strahlungsleistung Φ der wenigstens einen Strahlung emittierenden Strahlungsquelle (170) bewirkt durch Änderung wenigstens eines Parameters, gewählt aus der Gruppe Energie, Spannung, Strom, Frequenz der Lichtquelle und Kombinationen daraus, in Koordination mit einem Bewirken von Änderungen der Strahlungsleistung Φ wenigstens einer weiteren Strahlung emittierenden Strahlungsquelle (170), vorzugsweise von einigen anderen oder einer Gruppe von anderen Strahlung emittierenden Lichtquellen (170, 170) durch die Prozessor-Einheit der Vorrichtung (100), in Abhängigkeit von den Entfernungen d, d der Quelle(n) von einer jeweiligen ausgewählten Position innerhalb des Innenraums (130) der Vorrichtung (100).

## Revendications

1. Dispositif (100) pour une application de radiation actinique dirigée à un sujet (200), ledit dispositif (100) comprenant :
- un tunnel d'exposition (110) étant apte à entourer ledit sujet (200) devant être exposé à ladite radiation actinique, ledit tunnel d'exposition (110) étant formé d'une première surface (120) en forme de baril semi-cylindrique constituée d'un matériau sensiblement perméable à ladite radiation actinique devant être appliquée au sujet (200), et d'au moins une deuxième surface (140) constituée dudit matériau sensiblement perméable à ladite radiation actinique devant être appliquée au sujet (200), ladite au moins une deuxième surface (140) étant apte à compléter la première surface (120) dans la formation dudit tunnel d'exposition (110) ;
- les surfaces (120, 140) dudit tunnel d'exposition (110) séparant un espace intérieur (130) où un sujet (200) est exposé à ladite radiation actinique, depuis des espaces extérieurs (150, 160) où des sources (170) de lumière aptes à émettre une radiation actinique à travers lesdites surfaces (120, 140) sont montées ;
- lesdits espaces extérieurs (150, 160) étant logés dans au moins des première et deuxième parties d'un châssis (180, 190) du dispositif (100) et comprennent des moyens pour maintenir fixes, et opérer électriquement et électroniquement, lesdites sources (170) de lumière ; des moyens (182, 192) alimentant lesdites au moins des première et deuxième parties du châssis (180, 190) avec une énergie électrique ; des moyens aptes à ventiler, chauffer et/ou refroidir le dispositif (100); et des moyens (184, 194) pour l'opération du dispositif (100), incluant une unité de traitement ;
ledit dispositif (100) comprenant en outre
- au moins un moyen (171) apte à commander, et facultativement à faire varier, un flux radiant Φ d'au moins une source (170) de lumière émettant une radiation actinique en changeant des paramètres électroniques de l'au moins une source de lumière en fonction de la position géométrique de ladite au moins une source (170) de lumière émettant une radiation actinique, par rapport à sa distance à l'espace intérieur (130), obtenant ainsi une radiance E régulière au niveau du sujet (200) exposé à ladite radiation actinique,
dans lequel ledit moyen (171) apte à commander, et facultativement à faire varier, un flux radiant Φ d'au moins une source (170) de lumière émettant une radiation actinique comprend au moins un ou plus d'un élément électronique de commande (171) changeant les paramètres électroniques des sources de lumière en fonction de la position géométrique de ladite au moins une source (170) de lumière émettant une radiation actinique, par rapport à sa distance à l'espace intérieur (130).

2. Dispositif (100) selon la revendication 1, le dispositif étant configuré pour irradier un être humain devant être exposé à ladite radiation actinique dirigée.

3. Dispositif (100) selon la revendication 1 ou la revendication 2, dans lequel ledit tunnel d'exposition (110) a un axe longitudinal agencé horizontalement ou dans lequel ledit tunnel d'exposition (110) a un axe longitudinal agencé verticalement.

4. Dispositif (100) selon l'une quelconque des revendications 1 à 3, dans lequel ledit tunnel d'exposition (110) est formé de ladite surface (120) en forme de baril semi-cylindrique et d'au moins une surface (122) sensiblement plate, ou dans lequel ledit tunnel d'exposition (110) est formé de ladite surface (120) en forme de baril semi-cylindrique et d'une deuxième surface (121) en forme de baril semi-cylindrique.

5. Dispositif (100) selon la revendication 4, dans lequel ledit tunnel d'exposition (110) comprend au moins une surface (143) additionnelle en arc de cercle ou sensiblement plate, qui est préférablement également constituée d'un matériau sensiblement perméable à ladite radiation actinique devant être appliquée au sujet (200).

6. Dispositif (100) selon l'une quelconque des revendications 1 à 5, dans lequel, si ledit tunnel d'exposition (110) a un axe longitudinal agencé horizontalement, des sources de lumières supérieures (170u) aptes à émettre une radiation actinique à travers ladite surface (120) vers le bas jusqu'au sujet (200) allongé et des sources de lumières inférieures (170l) aptes à émettre une radiation actinique à travers ladite surface (140) vers le haut jusqu'au sujet (200) allongé sont montées au-dessus de ladite surface (120) dans ledit espace extérieur (150) et au-dessous de ladite surface (140) dans ledit espace extérieur (160); ou, si ledit tunnel d'exposition (110) a un axe longitudinal agencé verticalement, des sources de lumières de côté avant (170fs) aptes à émettre une radiation actinique à travers ladite surface (120) vers l'arrière jusqu'au sujet (200) debout, et des sources de lumières de côté arrière (170bs) aptes à émettre une radiation actinique à travers ladite surface (140) vers l'avant jusqu'au sujet (200) debout sont montées sur les côtés extérieurs desdites surfaces (120, 140) dans lesdits espaces extérieurs (150, 160).

7. Dispositif (100) selon l'une quelconque des revendications 1 à 6, dans lequel, dans le cas où ledit tunnel d'exposition (110) comprend au moins une surface (143) additionnelle en arc de cercle ou sensiblement plate, des sources (170a) de lumière additionnelles aptes à émettre une radiation actinique à travers ladite surface (143) jusqu'au sujet (200) allongé ou debout sont montées sur le côté extérieur de ladite surface (143) dans lesdits espaces extérieurs (150, 160).

8. Dispositif (100) selon l'une quelconque des revendications 1 à 7, dans lequel chaque source (170) de lumière émettant une lumière actinique est prévue avec au moins un, préférablement avec un, élément (171) électronique de commande changeant les paramètres électroniques des sources de lumière en fonction de la position géométrique de ladite une source (170) de lumière émettant une radiation actinique, par rapport à sa distance à l'espace intérieur (130) et à l'enveloppe (210) de forme (semi-)cylindrique (théorique) entourant le sujet (200) positionné dans ledit espace intérieur (130) pour se voir appliqué la radiation actinique dirigée.

9. Dispositif (100) selon l'une quelconque des revendications 1 à 7, dans lequel un groupe de sources (170) de lumière émettant une radiation actinique, préférablement une pluralité de groupes de sources (170, 170) de lumière émettant une radiation actinique, est prévu avec au moins un, préférablement avec un ou une pluralité (d')élément(s) (171, 171) électronique(s) de commande changeant les paramètres électroniques du groupe de sources de lumière en fonction de la position géométrique dudit groupe d'au moins une source (170) de lumière émettant une radiation actinique, par rapport à sa distance à l'espace intérieur (130).

10. Dispositif (100) selon l'une quelconque des revendications 1 à 9, dans lequel le paramètre électronique de la/des source(s) de lumière changé par ledit au moins un élément électronique de commande est choisi parmi le groupe consistant en la puissance, la tension, le courant, la fréquence de la source de lumière et des combinaisons de ceux-ci.

11. Dispositif (100) selon l'une quelconque des revendications 1 à 10, dans lequel le changement du/des paramètre(s) électronique(s) de la/des source(s) de lumière est un changement résultant : d'une détermination de la position géométrique de ladite au moins une source (170) de lumière émettant une radiation actinique et de sa distance d à une position sélectionnée à l'intérieur de l'espace intérieur (130) du dispositif (100) ; d'une détermination du changement requis du flux radiant Φ de ladite au moins une source de radiation pour parvenir à une radiance E régulière requise à ladite distance d ; d'une génération d'un ou de plus d'un signal, par une unité de traitement du dispositif (100), apte à effectuer un changement du/des paramètre(s) électronique(s) de la/des source(s) de lumière en fonction de la distance d de la/des source(s) de lumière à l'espace intérieur (130); d'une transmission dudit/desdits signal/signaux de l'unité de traitement du dispositif (100) à au moins un élément (171) électronique de commande apte à faire varier le flux radiant Φ de ladite au moins une source (170) de radiation émettant une radiation ; dans lequel ledit au moins un élément (171) de commande effectue une variation du flux radiant Φ de ladite au moins une source (170) de radiation émettant une radiation, en changeant au moins un sélectionné parmi la puissance, la tension, le courant, la fréquence de la source de lumière et des combinaisons de ceux-ci, en coordination avec, par l'unité de traitement du dispositif (100), l'exécution de variations du flux radiant Φ d'au moins une autre source (170) de radiation émettant une radiation, préférablement de plusieurs autres, ou d'un groupe d'autres, sources (170, 170) de lumière émettant une radiation, en fonction des distances d, d de la/des source(s) à une position sélectionnée respective à l'intérieur de l'espace intérieur (130) du dispositif (100).

12. Procédé non thérapeutique pour utiliser un dispositif (100) appliquant une radiation actinique dirigée à un sujet (200), ledit procédé comprenant les étapes de
- prévision d'un dispositif (100) selon l'une quelconque des revendications 1 à 11 ; et
- utilisation d'au moins une des sources (170) de lumière émettant une radiation de lumière actinique du dispositif en commandant au moins un flux radiant Φ d'au moins une desdites sources (170) de lumière émettant une radiation actinique en changeant au moins un de ses paramètres électroniques en fonction de la position géométrique de ladite au moins une source (170) de lumière émettant une radiation actinique, par rapport à sa distance à l'espace intérieur (130) du dispositif (100), obtenant ainsi une radiance E régulière à l'exposition à ladite radiation actinique émise par ladite source (170) de lumière.

13. Procédé selon la revendication 12, comprenant l'utilisation de sources (170, 170) de lumière émettant une radiation de lumière actinique uniques, ou de groupes de, ou de la totalité des sources (170, 170) de lumière émettant une radiation de lumière actinique en commandant au moins un flux radiant Φ de ladite/desdites source(s) (170, 170) de lumière émettant une radiation actinique en changeant au moins un de ses/leurs paramètre(s) électronique(s) en fonction de la position géométrique de ladite/desdites source(s) (170, 170) de lumière émettant une radiation actinique, par rapport à sa/leur distance à l'espace intérieur (130) du dispositif (100) au moyen d'au moins un élément (171) de commande, obtenant ainsi une radiance E régulière à l'exposition à ladite radiation actinique émise par ladite/lesdites source(s) (170, 170) de lumière.

14. Procédé selon la revendication 12 ou la revendication 13, dans lequel le paramètre électronique de la/des source(s) de lumière choisi parmi le groupe consistant en la puissance, la tension, le courant, la fréquence de la source de lumière et des combinaisons de ceux-ci est changé par ledit au moins un élément (171) électronique de commande.

15. Procédé selon l'une quelconque des revendications 12 à 14, ledit procédé comprenant les étapes de changement du/des paramètre(s) électronique(s) de la/des source(s) de lumière en déterminant la position géométrique de ladite au moins une source (170) de radiation émettant une radiation et de sa distance d à une position sélectionnée à l'intérieur de l'espace intérieur (130) du dispositif (100); en déterminant le changement requis du flux radiant Φ de ladite au moins une source de radiation pour parvenir à une radiance E régulière requise à ladite distance d ; en générant un ou de plus d'un signal, par une unité de traitement du dispositif (100), apte à effectuer un changement du/des paramètre(s) électronique(s) de la/des source(s) de lumière en fonction de la distance d de la/des source(s) de lumière à l'espace intérieur (130) ; en transmettant ledit/lesdits signal/signaux de l'unité de traitement du dispositif (100) à au moins un élément (171) électronique de commande apte à faire varier le flux radiant Φ de ladite au moins une source (170) de radiation émettant une radiation ; faisant ainsi en sorte que ledit au moins un élément (171) de commande effectue une variation du flux radiant Φ de ladite au moins une source (170) de radiation émettant une radiation, en changeant au moins un sélectionné parmi la puissance, la tension, le courant, la fréquence de la source de lumière et des combinaisons de ceux-ci, en coordination avec, par l'unité de traitement du dispositif (100), l'exécution de variations du flux radiant Φ d'au moins une autre source (170) de radiation émettant une radiation, préférablement de plusieurs autres, ou d'un groupe d'autres, sources (170, 170) de lumière émettant une radiation, en fonction des distances d, d de la/des source(s) à une position sélectionnée respective à l'intérieur de l'espace intérieur (130) du dispositif (100).
